# EUROPEAN PATENT APPLICATION

(11) **EP 2 060 282 A1**
(43) Date of publication of application: **20.05.2009**
(21) Application number: 07021989.4
(22) Date of filing: 13.11.2007
(51) Int. Cl.: A61M 1/10

(54) **Heart supporting device**

(71) Applicant: Al-Harbi, Abdulrahman Futayn, Tabuk (SA)
(72) Inventor: Al-Harbi, Abdulrahman Futayn, Tabuk (SA)
(74) Representative: Rau, Manfred

(57) **Abstract**

A heart supporting device (1) comprises an unstable magnet (3) which is disposed between a first stable magnet (2) and a second stable magnet (2). The polarity of the unstable magnet (3) can be changed such that attractive forces and repulsive forces can be generated between the stable magnets (2) and the unstable magnet (3) depending on the polarity of the unstable magnet (3). The magnets 2, 3 are fixed to an outer surface of a patient's heart such that the attractive forces result in a constrained contraction and the repulsive forces result in a constrained relaxation of the heart.

## Description

The invention relates to a heart supporting device.

The human heart is a muscular organ responsible for pumping blood through the blood vessels by repeated, rhythmic contractions. Deoxygenated blood is pumped via the muscle of the right ventricle into the lungs so that carbon dioxide can be dropped off and oxygen can be picked up. The oxygenated blood flows from the lungs to the left ventricle. The muscle of the left ventricle pumps the oxygenated blood out to the parts of the body. The right ventricle and the left ventricle are defined by the surrounding muscles creating the forces needed to pump the blood through the blood vessels.

Patients having a heart weakness suffer from a weak heart muscle. Either the muscle defining the right ventricle or the muscle defining the left ventricle or both muscles can be weak. Due to the lack of donor hearts for transplantation, patients are treated with heart supporting devices. These heart supporting devices are pumps that are integrated in the blood circulation and support a patient's heart in pumping the blood through the body. These known pumps have several disadvantages. Firstly, by the interaction with the blood main blood components, e.g. cells, plasma and platelets, are destroyed. Secondly, the pump fan generates blood turbulences inside the blood vessels. Thirdly, during the period of using the supporting pump the blood viscosity should be increased affecting the coagulation time.

It is an object of the invention to provide a simple heart supporting device which supports a weak heart muscle in an effective and gentle way.

According to the invention, this object is attained by the features of claim 1. By disposing the unstable magnet between the first stable magnet and the second stable magnet, attractive forces as well as repulsive forces can be generated between the unstable magnet and the stable magnets depending on the polarity of the unstable magnetic poles of the unstable magnet. The polarity of the unstable magnetic poles is controlled via the control unit. If the polarity of the unstable magnetic poles is such that the unstable magnetic poles are oriented in a direction that is opposite to that of the first and second stable magnetic poles, attractive forces are generated between the unstable magnet and the stable magnets. Due to the fixation of the magnets to the outer surface of the heart via fixation means, these attractive forces result in a constrained contraction of the heart muscle supporting the heart in its pumping function. If the polarity of the unstable magnetic poles is such that the unstable magnetic poles are co-oriented with the stable magnetic poles, repulsive forces are generated. Due to the fixation of the magnets to the outer surface of the heart by fixation means, these repulsive forces result in a constrained relaxation of the heart muscle. The relaxation allows the heart muscle to fill with blood again in order to start a new pumping cycle by changing the polarity of the unstable magnetic poles again and generating attractive forces between the unstable magnet and the stable magnets. Due to the fact that the heart supporting device supports the weak heart from an external side, the heart supporting device has no contact with the patient's blood. Thus, the disadvantages of conventional supporting pumps can be completely avoided.

Permanent magnets according to claim 2 are simple constructions of the first and second stable magnets.

An electromagnet according to claim 3 is a simple construction of the unstable magnet.

An iron core according to claim 4 increases the attractive forces and the repulsive forces between the unstable magnet and the stable magnets.

An electrical energy source according to claim 5 provides in a simple way the magnetising current which generates a magnetic field by flowing through the coil.

A frequency generating means according to claim 6, in particular frequency inverter, provides in a simple way an alternating current in order to operate the unstable magnet with a desired heart supporting frequency. The alternating current results in an alternating magnetic field of the unstable magnet such that the polarity of the unstable magnetic poles changes periodically corresponding to the alternating current. The alternating polarity of the unstable magnetic poles results in an alternation of attractive and repulsive forces such that the heart contracts and relaxes with the desired heart supporting frequency.

A measurement means according to claim 7 allows the monitoring of the patient's blood pressure.

An embodiment according to claim 8 allows to adapt the heart supporting frequency depending on the measured blood pressure. If the patient's blood pressure is too low, the control unit increases the heart supporting frequency via the electrical energy source. If the patient's blood pressure is too high, the control unit reduces the heart supporting frequency via the electrical energy source. Thus, the patient's blood pressure is corrected automatically.

Further features, advantages and details of the invention will become apparent from the ensuing description of an embodiment, taken in conjunction with the drawing, in which
- Fig. 1: is a first schematic view of a heart supporting device in a first operating condition resulting in a contraction of the heart mus- cle,
- Fig. 2: is a schematic view of the heart supporting device in a second operating condition resulting in a relaxation of the heart muscle,
- Fig. 3: is a schematic view of the heart supporting device fixed to the heart muscle in the first operating condition, and
- Fig. 4: is a schematic view of the heart supporting device fixed to the heart in the second operating condition.

A heart supporting device 1 comprises a plurality of stable magnets 2 and a plurality of unstable magnets 3. Each unstable magnet 3 is disposed between a first stable magnet 2 and second stable magnet 2.

In the following the arrangement of one unstable magnet 3 disposed between a first stable magnet 2 and a second stable magnet 2 is described in detail. The first stable magnet 2 has a first stable north pole 4 and a first stable south pole 5. The first stable north pole 4 and the first stable south pole 5 are in the following referred to as first stable magnetic poles 4, 5.

Correspondingly, the second stable magnet 2 has a second stable north pole 6 and a second stable south pole 7. The second stable north pole 6 and the second stable south pole 7 are in the following referred to as second stable magnetic poles 6, 7. The second stable magnetic poles 6, 7 are co-oriented with the first stable magnetic poles 4, 5. This means that the second stable north pole 6 is opposed to the first stable north pole 4 and the second stable south pole 7 is opposed to the first stable south pole 5. The first and the second stable magnets 2 are permanent magnets.

The unstable magnet 3 is an electromagnet comprising a coil 8 and an iron core 9. The coil 8 consists of a helical wire that is wound around the iron core 9 such that the iron core 9 is disposed inside the coil 8. The unstable magnet 3 has an unstable north pole 10 and an unstable south pole 11. The unstable north pole 10 and the unstable south pole 11 are in the following referred to as unstable magnetic poles 10, 11.

The coil 8 of the unstable magnet 3 is connected to an electrical energy source 12. The electrical energy source 12 comprises a direct current source 13 and a frequency generating means 14 in form of a frequency inverter. The frequency generating means is in the following referred to as frequency inverter 14. The direct current source 13 is connected to the frequency inverter 14. The frequency inverter 14 is connected to the coil 8 of the unstable magnet 3 such that a magnetising current I*_{M}* can flow through the wire of the coil 8. The frequency inverter 14 is designed such that the provided magnetising current I *_{M}* has essentially a sinusoidal form. Moreover, the frequency inverter 14 is designed such that a magnitude I and a heart supporting frequency F of the sinusoidal magnetising current I*_{M}* can be adjusted as desired.

The heart supporting device 1 further comprises a control unit 15 which is connected to the unstable magnet 3 via the electrical energy source 12. The control unit 15 is further connected to a measurement means 16 for measuring a patient's blood pressure. The control unit 15 is designed such that the heart supporting frequency F can be changed depending on the blood pressure measured by the measurement means 16.

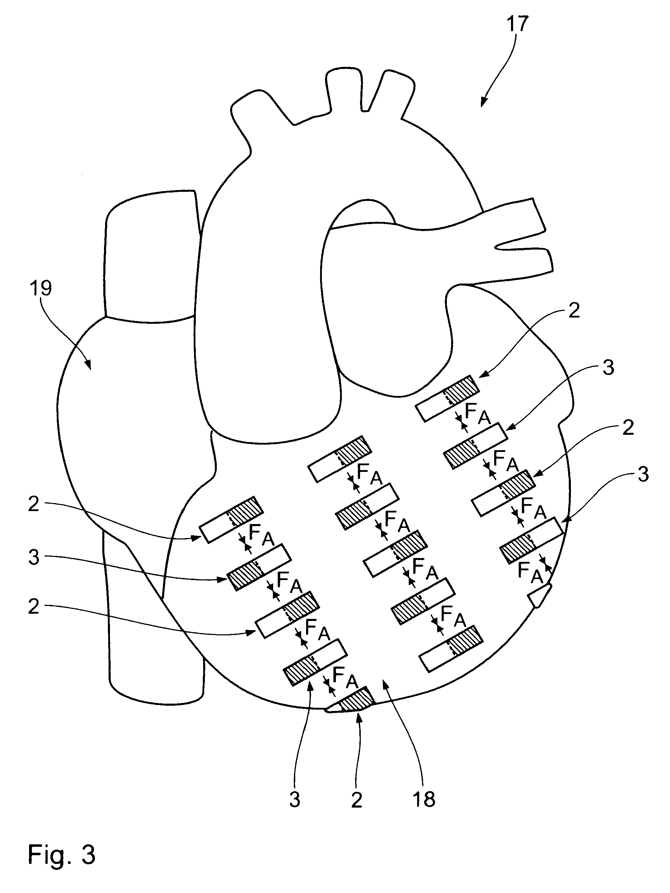

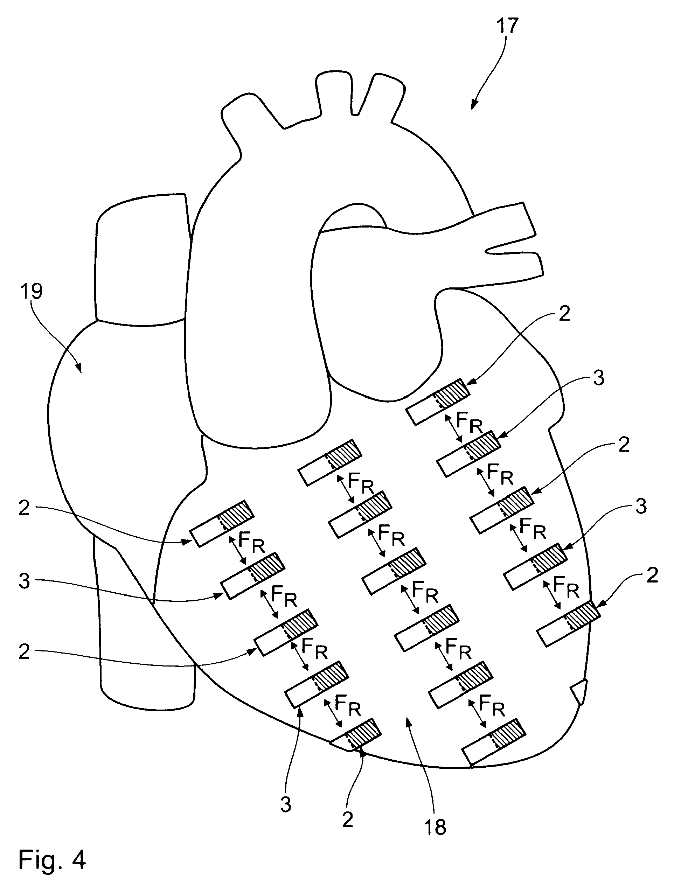

The stable magnets 2 and unstable magnets 3 are attached in ranks to a patient's heart 17 by fixation means. If the muscle of the right ventricle 18 is weak, the magnets 2, 3 are fixed to the outer surface of this muscle. This is shown in Figs. 3 and 4. Correspondingly, if the muscle of the left ventricle 19 is weak, the magnets 2, 3 are fixed to the outer surface of this muscle. Suitable fixation means are surgical sutures or suture clips.

The functionality of the heart supporting device 1 is as follows:
The direct current source 13 provides a direct current. This direct current is converted via the frequency inverter 14 into the magnetising current I*_{M}*. The magnetising current I*_{M}* is an alternating sinusoidal current. The magnitude I and the heart supporting frequency F of the magnetising current I*_{M}* can be controlled via the control unit 15.

The magnetising current I*_{M}* flows through the coils 8 of the unstable magnets 3 and generates magnetic fields such that the iron cores 9 are magnetised. The magnetised iron cores 9 amplify the magnetic fields of the unstable magnets 3. By designing the unstable magnets 3 as electromagnets, the magnetic flux density, the polarity of the unstable magnetic poles 10, 11 and the shape of the magnetic field can be controlled. The magnetic flux density is controlled by the magnitude I of the magnetising current I*_{M}*, the polarity of the unstable magnetic poles 10, 11 is controlled by the direction of the magnetising current I*_{M}* and the shape of the magnetic field is controlled by the shape of the iron core 9.

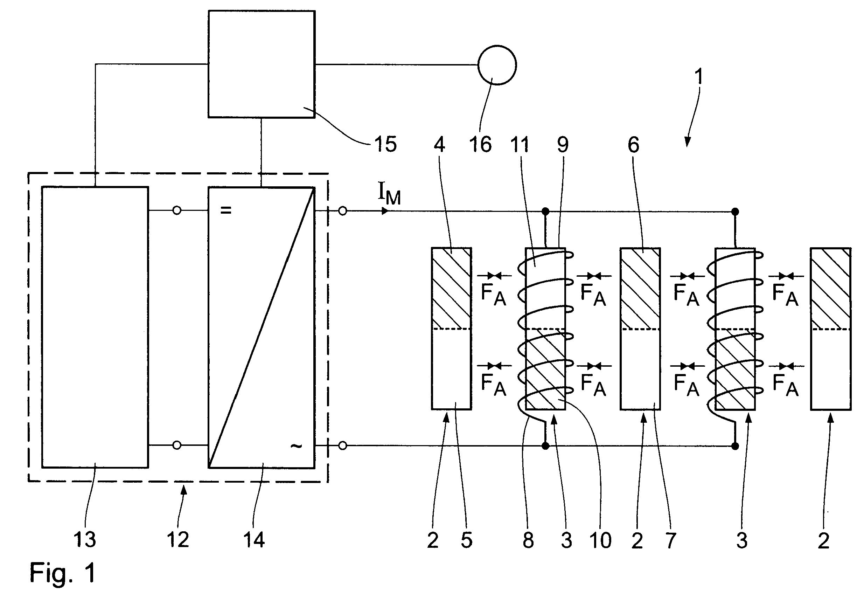

In a first operation condition, the direction of the magnetising current I*_{M}* is such that the stable magnetic poles 4, 5, 6, 7 and the unstable magnetic poles 10, 11 have an opposed orientation. This first operation condition is shown in Figs. 1 and 3. The first operation condition corresponds to the first half cycle of the magnetising current I*_{M}*. Due to the opposed orientation of the stable magnets 2 and the unstable magnets 3, attractive forces F*_{A}* are generated between each unstable magnet 3 and its adjacent stable magnets 2. The attractive forces F*_{A}* result in a constrained contraction of the muscle of the right ventricle 18. Thus, the heart 17 is supported in its pumping function. The magnitudes of the attractive forces F*_{A}* are controlled via the magnitude I of the magnetising current I*_{M}*.

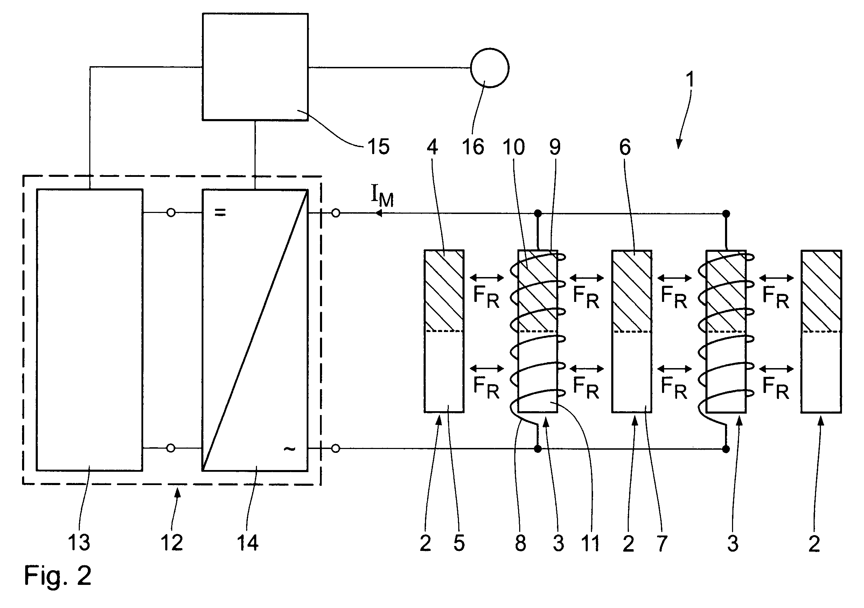

In a second operation condition, the direction of the magnetising current I*_{M}* is such that the stable magnetic poles 4, 5, 6, 7 and the unstable magnetic poles 10, 11 are co-oriented. This second operation condition is shown in Figs. 2 and 4. Due to the same orientation of the stable magnetic poles 4, 5, 6, 7 and the unstable magnetic poles 10, 11, repulsive forces F*_{R}* are generated between each unstable magnet 3 and its adjacent stable magnets 2. The repulsive forces F*_{R}* result in a constrained relaxation of the muscle of the right ventricle 18. This relaxation allows the right ventricle 18 to fill again with blood. The magnitudes of the repulsive forces F*_{R}* are controlled via the magnitude I of the magnetising current I*_{M}.* The second operation condition corresponds to the second half cycle of the magnetising current I*_{M}*.

Due to the sinusoidal form of the magnetising current I*_{M}*, the polarity of the unstable magnetic poles 10, 11 changes periodically as the magnetising current I*_{M}* reverses its direction every half cycle. Thus, attractive forces F*_{A}* and repulsive forces F*_{R}* are generated corresponding to the heart supporting frequency F of the magnetising current I*_{M}*.

The heart supporting frequency F is controlled automatically via the measured blood pressure. If the blood pressure is too low, the control unit 15 controls the frequency inverter 14 such that the heart supporting frequency F is increased. The increased heart supporting frequency F results in an increased blood pressure. If the measured blood pressure is too high, the control unit 15 controls the frequency inverter 14 such that the heart supporting frequency F is reduced. The reduced heart supporting frequency F results in a reduced blood pressure. Thus, the blood pressure is corrected automatically via the control unit 15, the measurement means 16 and the electrical energy source 12.

## Claims

1. A heart supporting device, comprising
a. a first stable magnet (2) with first stable magnetic poles (4, 5),
b. a second stable magnet (2) with second stable magnetic poles (6, 7), the second stable magnetic poles (6, 7) being co-oriented with the first stable magnetic poles (4, 5),
c. an unstable magnet (3) with unstable magnetic poles (10, 11), the unstable magnet (3) being disposed between the first stable magnet (2) and the second stable magnet (2),
d. fixation means for fixing the magnets (2, 3) to an outer surface of a heart (17), and
e. a control unit (15) being designed such that a polarity of the unstable magnetic poles (10, 11) is changeable.

2. A heart supporting device according to claim 1, wherein the first and the second stable magnets (2) are permanent magnets.

3. A heart supporting device according to claim 1 or 2, wherein the unstable magnet (3) is an electro magnet with a coil (8).

4. A heart supporting device according to claim 3, wherein the unstable magnet (3) comprises an iron core (9), the coil (8) being wound around the iron core (9).

5. A heart supporting device according to claim 3 or 4, wherein the heart supporting device (1) comprises an electrical energy source (12), the electrical energy source (12) being connected to the coil (8) of the unstable magnet (3).

6. A heart supporting device according to claim 5, wherein the electrical energy source (12) comprises a frequency generating means (14) for operating the unstable magnet (3) with a heart supporting frequency (F).

7. A heart supporting device according to one of the claims 1 to 7, wherein the heart supporting device (1) comprises a measurement means (16) for measuring a blood pressure, the measurement means (16) being connected with the control unit (15).

8. A heart supporting device according to claim 7, wherein the electrical energy source (12) is connected to the control unit (15) such that the heart supporting frequency (F) is changeable depending on the measured blood pressure.
